# EUROPÄISCHE PATENTSCHRIFT

(11) **EP 2 460 471 B1**
(45) Veröffentlichungstag und Bekanntmachung des Hinweises auf die Patenterteilung: **03.07.2013**
(21) Anmeldenummer: 10015297.4
(22) Anmeldetag: 04.12.2010
(51) Int. Cl.: A61B 5/151

(54) **Lanzettenvorrichtung mit optional wiederverwendbaren magazinierten Lanzetten**
Lancet device with optionally reusable stored lancets
Dispositif de lancettes doté de lancettes logées optionnellement réutilisables

(43) Veröffentlichungstag der Anmeldung: 06.06.2012
(73) Patentinhaber: Roche Diagnostics GmbH, 68298 Mannheim (DE); F. Hoffmann-La Roche AG, 4070 Basel (CH)
(72) Erfinder: Kuhr, Hans-Jürgen, 68219 Mannheim (DE); List, Hans, 64754 Hesseneck-Kailbach (DE); Keil, Michael, 67071 Ludwigshafen (DE)

(56) Entgegenhaltungen:
- DE-B3- 10 326 692
- US-A1- 2007 016 104
- US-A1- 2010 168 616

## Beschreibung

Die vorliegende Erfindung betrifft eine Lanzettenvorrichtung zum Erzeugen einer Einstichwunde, insbesondere zur Gewinnung einer Probe einer Körperflüssigkeit, das eine Magazinaufnahme für ein Lanzettenmagazin mit einer Mehrzahl von Lanzetten aufweist. Die erfindungsgemäße Lanzettenvorrichtung kann wahlweise so betrieben werden, dass entweder durch automatisches Weitertakten des Lanzettenmagazins für jeden Einstichvorgang eine neue Lanzette eingesetzt wird oder das automatische Weitertakten des Lanzettenmagazins unterdrückt werden kann, sodass mit der zuletzt verwendeten Lanzette gewünschtenfalls mehrere Einstiche durchgeführt werden können, bevor zum gewünschten Zeitpunkt eine ungebrauchte Lanzette zum Einsatz kommt.

Die vorliegende Erfindung betrifft darüber hinaus ein Entnahmesystem zur Entnahme einer Probe einer Körperflüssigkeit umfassend eine wie vorstehend genannte Lanzettenvorrichtung und ein dazu passendes Lanzettenmagazin mit mehreren Lanzetten.

Um für analytische oder diagnostische Zwecke eine geringe Menge einer Körperflüssigkeit wie z. B. Blut aus einem geeigneten Körperteil wie beispielsweise aus der Fingerbeere oder dem Ohrläppchen zu entnehmen, werden üblicherweise Lanzetten verwendet, die zur Erzeugung einer Einstichwunde in das entsprechende Körperteil gestochen werden können. Zur entsprechenden Anwendung geeignete Blutentnahmesysteme, die aus einer Lanzettenvorrichtung und daran angepasste Lanzetten bestehen, sind hinlänglich bekannt. Üblicherweise umfassen derartige Lanzettenvorrichtungen ein Gehäuse, in dem sich ein Lanzettenantrieb befindet, der die Lanzette mechanisch zum Einstich in gewählte Hautpartie bewegt.

An Lanzettenvorrichtungen werden heutzutage hohe Anforderungen bezüglich der Erzeugung eines minimalen Schmerzempfindens einerseits, sowie einfacher Bedienbarkeit, kompakter bauweise sowie kostengünstiger Herstellung und wirtschaftlichen Betriebskosten andererseits gestellt. Im Fordergrund steht dabei eine einfache und komfortable Bedienung der Lanzettenvorrichtung, die insbesondere für Diabetiker wichtig ist, die ihren Blutzuckerspiegel häufig und regelmäßig durch Selbstkontrolle bestimmen müssen. Dabei führt eine einfache Bedienbarkeit und eine geringe Schmerzbelastung häufig zu einer höheren Bereitschaft der Patienten, diesem Erfordernis folge zu leisten und somit den Therapieerfolg zu unterstützen.

Ein bedeutender Fortschritt in dieser Hinsicht stellt die Verwendung von magazinierten, d.h. in einem Magazin enthaltenen Lanzetten dar. Derartige Lanzettenmagazine enthalten eine Mehrzahl von Lanzetten und können in die Magazinaufnahme einer entsprechenden Lanzettenvorrichtung eingesetzt werden. Dadurch erübrigt sich häufiges Einsetzen bzw. Austauschen einzelner Lanzetten, was insbesondere für Diabetespatienten mit eingeschränkter Tast- oder Sehfähigkeit eine deutliche Erleichterung darstellt. Dementsprechend erhöht sich die Nachfrage nach Lanzettenvorrichtungen mit magazinierten Lanzetten stetig. Der Einsatz von magazinierten Lanzetten bzw. Lanzettenmagazinen in Lanzettenvorrichtungen erfordert in der Regel eine Mechanik zum Fortschalten bzw. Weitertakten des Magazins mittels der bewerkstelligt werden kann, dass im Anschluss an einen Einstichvorgang eine unbenutzte Lanzette zum Einsatz kommen kann. Es hat sich allerdings gezeigt, dass es auch bei Verwendung von magazinierten Lanzetten und den entsprechenden Lanzettenvorrichtungen insbesondere aus ökonomischen Erwägungen heraus auch wünschenswert sein kann, magazinierte Lanzette gewünschtenfalls wiederholt zu benutzen.

Dementsprechend wird in WO 2006/027101 A1 eine Lanzettenvorrichtung beschrieben, die ein Lanzettenmagazin mit mehreren Lanzetten aufnehmen kann und die eine Magazinfortschaltung aufweist, mit der das Lanzettenmagazin so fortschaltbar ist, dass die in ihm gelagerten Lanzetten nacheinander mit einer Schubstange des Lanzettenantriebs kuppelbar sind, wobei der Lanzettenantrieb mechanisch von der Magazinfortschaltung entkoppelt ist, und wobei die Lanzettenvorrichtung ein Betätigungselement aufweist, mit dem die Magazinfortschaltung unabhängig von dem Lanzettenantrieb, insbesondere unabhängig von der Spanneinrichtung, betätigbar ist. Dadurch steht es dem Anwender jederzeit frei, durch Betätigung der Magazinfortschaltung eine bereits gebrauchte gegen eine ungebrauchte Lanzette auszuwechseln. Als nachteilig ist dabei allerdings zu betrachten, dass die Magazinfortschaltung in jedem Falle ein aktives Handeln des Benutzers erfordert, was dazu führen kann, dass eine bereits benutzte Lanzette durch Vergessen des Weitertaktens unerwünscht oft zum Einsatz kommen kann.

Die US 2006/0161078 A1 offenbart eine kreisscheibenförmige Kassette für ein Testgerät sowie ein Testverfahren unter Einsatz des genannten Testgerätes mit der Kassette. Die Kassette umfasst sowohl eine Mehrzahl von Lanzettennadeln als auch eine Mehrzahl von Teststreifen und einen Mechanismus zur Verknüpfung mit dem Testgerät. Da jedes Testelement nur einmal verwendet werden kann, muss die Kassette im Anschluss an einen durchgeführten Testvorgang bzw. Einstich manuell oder automatisch, z.B. mittels eines elektrischen Antriebs, weitergetaktet werden.

In der US 2007/0299458 A1 werden ebenfalls spezielle Stechhilfen mit einem kreisscheibenförmigen Lanzettenmagazin offenbart, wobei die Lanzetten jeweils in Einstichrichtung radial nach außen angeordnet sind. Die Stechhilfen weisen einen Mechanismus auf, der die beabsichtigte oder unbeabsichtigte Wiederbenutzung einer bereits gebrauchten Lanzette ausschließt. Dies wird dadurch bewerkstelligt, das bei jedem Spannvorgang des Lanzettenantriebs das Lanzettenmagazin weitergedreht wird, so dass eine noch unbenutzte Lanzette mit dem Lanzettenantrieb in Verbindung tritt und darüber hinaus die austauschbaren Lanzettenmagazine eine Sperre zum Verhindern eines Wiedereinlegens benutzter Magazine aufweisen.

Die WO 2009/067269 A1 betrifft eine Stechhilfe mit einem auswechselbaren kreischeibenförmigen Magazin, das eine Mehrzahl von Lanzetten umfasst. Die Vorrichtung weist eine Rückdrehsperre auf, sodass das Magazin nur in eine Richtung weitergetaktet werden kann und somit eine erneute Benutzung einer bereits gebrauchten Lanzette verhindert ist. Darüber hinaus wird ein Mechanismus offenbart, der es ermöglicht, eine oder mehrere der zuvor bereits benutzten Lanzetten im Notfall erneut zu benutzen. Um eine versehentliche oder missbräuchliche Nutzung vorzubeugen, muss dazu das aufgebrauchte Lanzettenmagazin gegebenenfalls aus der Stechhilfe herausgenommen, jedenfalls jedoch manuell entgegen der eigentlichen Drehrichtung zurückgedreht werden.

Die US 201010168616 A1 offenbart ein trommelförmiges Magazin mit einer Mehrzahl drin angeordneter Lanzetten sowie einer Mehrzahl zugehöriger Federn. Das trommelförmige Magazin kann von einem Benutzer manuell mittels eines "arming member" in vorbestimmtem Drehsinn rotiert werden.

DE 103 26 692 B3 betrifft ein Blutentnahmesystem mit Gehäuse, Lanzettenführung, Lanzettenantrieb und Lanzetten-Vorratsbehältnis, das dadurch gekennzeichnet ist, dass es ein Lanzettenspitzen-Schutzelement umfasst, in das die Lanzettenspitze vor oder nach einer Einstechbewegung einführbar ist, wobei das Schutzelement eine darin eingeführte Lanzettenspitze mechanisch und hygienisch schützt, und dass das Lanzettenspitzen-Schutzelement in einer Parkposition der Lanzetten auf der Lanzettenspitze angeordnet ist, wobei die Parkposition nicht mit der Entnahmeposition und der Stechposition übereinstimmt.

Ausgehend von diesem Stand der Technik bestand die der vorliegenden Erfindung zu Grund liegende Aufgabe darin, eine Lanzettenvorrichtung bereitzustellen, die die Entnahme einer geringen Menge einer Körperflüssigkeit, vorzugsweise Blut ermöglicht sowie die jeweiligen Nachteile des Standes der Technik umgeht und dabei
- eine möglichst gute Bedienbarkeit aufweist,
- einen möglichst hohen Schutz vor ungewünschten Begleiterscheinungen wie z.B. Infektionen bietet,
- gewünschtenfalls die wiederholte Verwendung bereits gebrauchter Lanzetten ermöglicht,
- dabei jedoch gewährleistet, dass nur die jeweils zuletzt benutzte Lanzette wieder verwendet werden kann und
- bei Bedarf unabhängig von der Verfügbarkeit von Verbrauchsmitteln wie unbenutzten Lanzettenmagazinen einsetzbar ist.

Die Aufgabe wurde erfindungsgemäß gelöst durch die Bereitstellung einer Lanzettenvorrichtung zum Erzeugen einer Einstichwunde, umfassend
- eine Magazinaufnahme für ein Lanzettenmagazin mit mehreren Lanzetten;
- einen Lanzettenantrieb mit einer Antriebsfeder, einer Spanneinrichtung zum Spannen der Antriebsfeder und einem Verbindungselement, das mit einer in dem Lanzettenmagazin angeordneten Lanzette in Kontakt gebracht werden kann und das mittels des Lanzettenantriebs die mit ihm in Kontakt gebrachte Lanzette zu einer Einstichbewegung bewegen kann um eine Einstichwunde zu erzeugen;
- eine Magazinfortschaltung, mit der das Lanzettenmagazin so fortschaltbar ist, dass die in ihm gelagerten Lanzetten nacheinander mit dem Verbindungselement in Kontakt gebracht werden können und die einen aktiven und einen passiven Betriebszustand aufweist und
- ein Wahlelement, mit dem die Magazinfortschaltung wahlweise in den aktiven oder in den passiven Betriebszustand versetzt werden kann,
wobei im aktiven Betriebszustand die Magazinfortschaltung das Lanzettenmagazin vor oder nach einer Einstichbewegung einer Lanzette automatisch fortschaltet, so dass bei der darauf folgenden Einstichbewegung eine noch unbenutzte Lanzette des Lanzettenmagazins mit dem Verbindungselement des Lanzettenantriebs in Kontakt gebracht wird und wobei im passiven Betriebszustand die automatische Magazinfortschaltung deaktiviert ist, so dass vor oder nach einer Einstichbewegung einer Lanzette das Lanzettenmagazin nicht fortgeschaltet wird, so dass bei der darauf folgenden Einstichbewegung die gleiche Lanzette wie bei der vorherigen Einstichbewegung erneut mit dem Verbindungselement des Lanzettenantriebs in Kontakt gebracht wird.

Die vorliegende Erfindung betrifft in einem Aspekt eine Lanzettenvorrichtung zum Erzeugen einer Einstichwunde die sich zur Gewinnung von kleinvolumigen Proben einer Körperflüssigkeit für Diagnosezwecke. Als Beispiele für derartige Körperflüssigkeiten seien Blut oder interstitielle Flüssigkeit, bevorzugt aber Blut genannt. Die erfindungsgemäße Lanzettenvorrichtung, oft auch als Stechhilfe bezeichnet, eignet sich zum mehrmaligen Gebrauch und weist eine Magazinaufnahme für ein Lanzettenmagazin mit mehreren Lanzetten auf.

Das erfindungsgemäß zur Einbringung in die Magazinaufnahme vorgesehen Lanzettenmagazin eignet sich zur Aufnahme und Lagerung einer Mehrzahl, üblicherweise 2 bis 50, in der Regel 2 bis 25, bevorzugt 3 bis 10, besonders bevorzugt 4 bis 8 und insbesondere bevorzugt von 6 bis 8, am meisten bevorzugt von 6 Lanzetten. Das Magazin umfasst üblicherweise ein Magazingehäuse, das die genannte Mehrzahl von Lanzetten zumindest teilweise umgibt und mindestens eine Austrittsöffnung zum Austritt der jeweiligen Lanzettenspitze aufweist. Bevorzugt weist das Lanzettenmagazin einen segmentierten Aufbau auf, wobei jeweils eine Lanzette bevorzugt räumlich getrennt von den weiteren Lanzetten in einem Segment, im Folgenden auch als Lanzettenaufnahme bezeichnet, gelagert ist. In dieser Ausführung ist es vorteilhaft, wenn jedes der genannten Segmente eine eigene Austrittöffnung für die darin gelagerten Lanzetten aufweist.

Die räumliche Ausgestaltung des genannten Lanzettenmagazins unterliegt keinen grundsätzlichen Beschränkungen, sollte sich jedoch vorteilhaft an der Ausgestaltung bzw. Form der entsprechenden Magazinaufnahme bzw. Lanzettenvorrichtung orientieren. Grundsätzlich können verschiedene, dem Fachmann geläufige Magazinformen im Rahmen der vorliegenden Erfindung eingesetzt werden, beispielsweise Trommelmagazine, Flachmagazine, Stapelmagazine, Bandmagazine, Gurtmagazine und dergleichen mehr. Vor diesem Hintergrund hat es sich als vorteilhaft erwiesen, wenn das erfindungsgemäß vorgesehene Lanzettenmagazin rotationssymmetrisch ausgestaltet ist, beispielsweise in Form einer Scheibe oder bevorzugt in Form einer Trommel.

Die genannten Lanzettenmagazine weisen dann eine Rotationsachse auf, die vorteilhafterweise parallel zur Geräteachse der Lanzettenvorrichtung verläuft oder weiterhin bevorzugt, insbesondere im Fall der vorstehend genannten Trommelmagazine sogar mit dieser identisch ist. Darüber hinaus kann es auch vorteilhaft sein, wenn die Rotationsachse im Wesentlichen senkrecht zur Geräteachse ausgerichtet ist, insbesondere eines wie vorstehend genannten Scheibenmagazins. Unter dem Begriff Geräteachse ist dabei eine Achse zu verstehen, die parallel zur Einstichrichtung der jeweiligen Lanzette verläuft und vorzugsweise durch das distale sowie das proximale Ende der Lanzettevorrichtung verläuft. Im Rahmen einer bevorzugten Ausführungsform handelt es sich bei dem in Form einer Trommel ausgebildeten Lanzettenmagazin um ein sogenanntes Revolvermagazin, bei dem die einzelnen Lanzetten jeweils parallel zur Rotationsachse des Trommelmagazins ausgerichtet sind. Die Mehrzahl von Lanzetten befindet sich dabei bevorzugt in ringförmig angeordneten Lanzettenaufnahmen des Lanzettenmagazins.

Die Magazinaufnahme der erfindungsgemäßen Lanzettenvorrichtung ist vorteilhaft so ausgestaltet, das ein wie vorstehend beschriebenes Lanzettenmagazin darin eingesetzt werden und mechanisch mit der erfindungsgemäßen Lanzettenvorrichtung verbunden werden kann. Neben der genannten Magazinaufnahme weist die erfindungsgemäße Lanzettenvorrichtung in der Regel noch ein Gehäuse mit einer Austrittsöffnung auf, wobei die Austrittsöffnung auch an einer zum Gehäuse zugehörigen Kappe vorgesehen sein kann. Das Gehäuse weist eine für den Benutzer von außen zugängliche Oberfläche auf, die im Folgenden auch als Bedienoberfläche bezeichnet wird. Im Rahmen einer weiterhin bevorzugten Ausführungsform umgibt die genannte Kappe ein in die Magazinaufnahme eingelegtes Lanzettenmagazin und kann somit die Magazinaufnahme verschließen. Die genannte Kappe kann darüber hinaus mit einem Mechanismus zum Einstellen der Einstechtiefe verbunden sein, beispielweise in drehbar gelagerter, über ein Gewinde mit dem restlichen Gehäuse verbundener Form.

Die erfindungsgemäße Lanzettenvorrichtung weist darüber hinaus einen Lanzettenantrieb mit einer Antriebsfeder, einer Spanneinrichtung zum Spannen der Antriebsfeder und einem Verbindungselement auf, wobei das Verbindungselement mit einer in dem Lanzettenmagazin angeordneten Lanzette in Kontakt gebracht werden kann und das mittels des Lanzettenantriebs die mit ihm in Kontakt gebrachte Lanzette zu einer Einstichbewegung bewegen kann um eine Einstichwunde zu erzeugen. Im Rahmen einer bevorzugten Ausführungsform ist das Verbindungselement als Schubstange ausgebildet, die mittels des Lanzettenantriebs zusammen mit einer daran gekoppelten Lanzette mit hoher Geschwindigkeit derartig bewegt werden kann, dass eine Einstichbewegung, bevorzugt eine Einstich- und Rückführbewegung ausgerührt werden kann. Ein derartiger Mechanismus ist in der WO 2006/027101 offenbart, auf die hiermit diesbezüglich Bezug genommen wird.

Die im Rahmen des Lanzettenantriebs vorgesehene Antriebsfeder kann in jeder dem Fachmann geeignet erscheinenden Form ausgestaltet sein, beispielsweise als Blattfeder oder als Spiralfeder, die beispielsweise durch Streckung, Stauchung oder Verdrillung gespannt werden kann. Die genannte Antriebsfeder wird mittel einer Spanneinrichtung gespannt, wobei die Spanneinrichtung üblicherweise ein Betätigungselement umfasst, dass mit der Spanneinrichtung so zusammen wirkt, dass die Antriebsfeder bei einer Betätigung desselben, vorzugsweise beim Niederdrücken des Betätigungselementes, gespannt wird.

Im Rahmen einer erfindungsgemäß ebenfalls bevorzugten Ausführungsform umfasst der Lanzettenantrieb einen durch die Antriebsfeder antreibbaren Antriebsrotor und einen abtriebseitigen Kupplungsmechanismus, durch den in der Vortriebsphase des Lanzettenantriebs eine Drehbewegung des Antriebsrotors in eine Translationsbewegung der Schubstange und über diese in eine Einstichbewegung der Lanzette umgesetzt wird.

Im Rahmen dieser erfindungsgemäß bevorzugten Ausführungsform schließt die Spanneinrichtung einen drehbewegliche Spannrotor ein, an dem sich das von dem Antriebsrotor abgewandte Ende der Antriebsfeder abstützt. Dieser Spannrotor ist zum Spannen der Antriebsfeder bei gehemmter Rotation des Antriebsrotors in die gleiche Drehrichtung drehbar, in die sich der Antriebsrotor während der Vortriebsphase dreht. Der Spannrotor ist während der Vortriebsphase gegen eine Rückwärtsdrehung arretiert, so dass der Antriebsrotor nach Freigabe der Hemmung eine Drehbewegung durchführt, die in eine Translationsbewegung der Schubstange umgesetzt wird. Der Antriebsrotor durchläuft vorteilhafterweise bei einem Arbeitszyklus einen Gesamtdrehwinkelbereich von 360°.

Die Spanneinrichtung wird im Rahmen dieser bevorzugten Ausführungsform über ein Drehschiebegetriebe betätigt, das mit einem Betätigungselement in der oben beschriebenen weise zusammenwirkt und vorteilhaft von einer Steuerkurve in Form einer umlaufenden Doppelnut auf dem Spannrotor und zwei Steuerzapfen der Spannhülse gebildet wird, von denen die Steuerkurve beim Niederdrücken des Betätigungselementes abgefahren wird. Ein derartiger Antriebsmechanismus ist in der EP 1 384 438 A1 offenbart, auf die hiermit diesbezüglich Bezug genommen wird. Die genannte Antriebsart stellt eine bevorzugte aus einer Vielzahl von möglichen Antriebsarten dar, die dem Fachmann geläufig sind und die er anstelle der dargestellten Antriebsart einsetzen kann.

Die erfindungsgemäße Lanzettenvorrichtung weist darüber hinaus eine Magazinfortschaltung auf, mit der das Lanzettenmagazin so fortschaltbar ist, dass die in ihm gelagerten Lanzetten nacheinander mit dem Verbindungselement in Kontakt gebracht werden können und die einen aktiven und einen passiven Betriebszustand aufweist. Unter dem Begriff Magazinfortschaltung ist dabei ein Mechanismus zu verstehen, mit dem im Anschluss an eine bereits benutzte Lanzette eine noch unbenutzte der Mehrzahl von im Lanzettenmagazin befindlichen Lanzetten mit dem Verbindungselement und somit mittelbar mit dem Lanzettenantrieb in Kontakt gebracht werden kann. Dieser Vorgang, bei dem eine bereits benutzte Lanzette durch eine noch unbenutzte Lanzette des gleichen Lanzettenmagazins derart ersetzt wird, dass die noch unbenutzte Lanzette in einer Position befindet, in der sie mit dem Verbindungselement in Kontakt gebracht bzw. bevorzugt an dieses angekuppelt werden kann, wird im Rahmen der vorliegenden Erfindung als Fortschalten oder auch als Weitertakten des Lanzettenmagazins bezeichnet. So könnte ein linear auf gebautes Lanzettenmagazin, bei dem die einzelnen Lanzetten neben- oder hintereinander angeordnet sind etwa durch Verschiebung fortgeschaltet werden. Im erfindungsgemäß bevorzugten Fall, bei dem die Mehrzahl von Lanzetten in einem wie vorstehend beschriebenen rotationssymmetrischen, beispielweise Scheiben- oder trommelförmigen Lanzettenmagazin entlang in radialer oder axialer Ausrichtung angeordnet sind, erfolgt ein Fortschalten bzw. Weitertakten durch Drehung um die Rotationsachse des Magazins, wobei der Drehwinkel beim Fortschalten in der Regel durch die Anzahl der Lanzetten im Magazin bzw. deren Abstand voneinander bestimmt wird.

Im Rahmen einer bevorzugten Ausführungsform betrifft die vorliegende Erfindung daher eine Lanzettenvorrichtung zum Erzeugen einer Einstichwunde, umfassend
- eine Magazinaufnahme für ein Lanzettenmagazin mit mehreren Lanzetten;
- einen Lanzettenantrieb mit einer Antriebsfeder, einer Spanneinrichtung zum Spannen der Antriebsfeder und einem Verbindungselement, das an eine in dem Lanzettenmagazin angeordnete Lanzette ankuppelbar ist und das mittels des Lanzettenantriebs zusammen mit einer daran angekuppelten Lanzette zu einer Einstichbewegung bewegt werden kann um eine Einstichwunde zu erzeugen;
- eine Magazinfortschaltung, mit der das Lanzettenmagazin so fortschaltbar ist, dass die in ihm gelagerten Lanzetten nacheinander mit dem Verbindungselement kuppelbar sind und die einen aktiven und einen passiven Betriebszustand aufweist und
- ein Wahlelement, mit dem die Magazinfortschaltung wahlweise in den aktiven oder in den passiven Betriebszustand versetzt werden kann,
wobei im aktiven Betriebszustand die Magazinfortschaltung das Lanzettenmagazin vor oder nach einer Einstichbewegung einer Lanzette automatisch fortschaltet, so dass bei der darauf folgenden Einstichbewegung eine noch unbenutzte Lanzette des Lanzettenmagazins mit dem Verbindungselement gekuppelt wird und wobei im passiven Betriebszustand die automatische Magazinfortschaltung deaktiviert ist, so dass vor oder nach einer Einstichbewegung einer Lanzette das Lanzettenmagazin nicht fortgeschaltet wird, so dass bei der darauf folgenden Einstichbewegung die gleiche Lanzette wie in der vorherigen Einstich- und Rückführbewegung erneut mit dem Verbindungselement gekuppelt wird.

Die genannte Magazinfortschaltung weist erfindungsgemäß einen aktiven und einen passiven Betriebszustand auf. Unter dem Begriff aktiver Betriebszustand ist dabei zu verstehen, dass die Magazinfortschaltung das Lanzettenmagazin vor oder nach einer Einstichbewegung einer Lanzette automatisch fortschaltet, so dass bei der darauf folgenden Einstichbewegung eine noch unbenutzte Lanzette des Lanzettenmagazins mit dem Verbindungselement des Lanzettenantriebs in Kontakt gebracht wird. Dabei handelt es sich bei einer automatischen Fortschaltung um eine solche, bei der zum Fortschalten des Lanzettenmagazins kein separater Bedienschritt durch den Benutzer der erfindungsgemäßen Lanzettenvorrichtung notwendig ist. Dies kann beispielsweise dadurch erreicht werden, dass die Magazinfortschaltung mechanisch mit dem Lanzettenantrieb gekoppelt ist. Diese Kopplung kann beispielsweise so ausgestaltet sein, dass beim Spannen des Lanzettenantriebs, etwa wie vorstehend beschrieben beim Niederdrücken des Betätigungselementes gleichzeitig das jeweils eingesetzte Magazin fortgeschaltet wird. Diese Form der automatischen Magazinfortschaltung wird beispielsweise in der WO 2003/071940 beschrieben. Alternativ kann beispielsweise auch die Restenergie der Stichbewegung zum Weitertakten des Magazins verwendet werden wie beispielsweise in der DE 10022720 A1 beschrieben. In beiden Fällen wären keine separaten Bedienschritte durch den Benutzer zum Weitertakten notwendig und es würde automatisch vor einem Einstich jeweils eine neue, noch unbenutzte Lanzette mit dem Verbindungselement des Lanzettenantriebs in Kontakt gebracht werden.

Unter dem Begriff "vor oder nach einer Einstichbewegung" ist im Zusammenhang mit dem Fortschalten des Magazins im Rahmen der vorliegenden Erfindung zu verstehen, dass das Fortschalten des Lanzettenmagazins entweder vor, bevorzugt unmittelbar vor dem Beginn der Einstichbewegung einer Lanzette erfolgt oder erst nach, bevorzugt unmittelbar nach dem Abschluss der Einstichbewegung erfolgt. Dabei beziehen sich die Begriffe vor bzw. nach auf den jeweiligen Bedienzyklus der Lanzettenvorrichtung, bei dem diese zunächst gespannt und dann ausgelöst wird. Im Anschluss daran erfolgt eine Einstichbewegung der jeweils mit dem Lanzettenantrieb in Kontakt gebrachten Lanzette der nach Erreichen der maximalen Auslenkung der Lanzette in Einstichrichtung in eine Rückführbewegung übergeht. Unter einer Fortschaltung nach einer Einstichbewegung kann dabei sowohl eine Fortschaltung des Magazins nach Abschluss der Einstich- und Rückführbewegung einer Lanzette verstanden werden als auch bevorzugt eine Fortschaltung unmittelbar nach einer Einstichbewegung. In letzterem Falle kann die Magazinfortschaltung auch ganz oder teilweise während der Rückführbewegung einer Lanzette erfolgen.

Darüber hinaus ist es auch im Rahmen der vorliegenden Erfindung vorteilhaft, wenn das jeweils eingesetzte Magazin, insbesondere ein eingesetztes Trommelmagazin nur so lange weitergeschaltet werden kann, bis die letzte noch unbenutzte Lanzette mit dem Verbindungselement des Lanzettenantriebs in Kontakt gebracht, d.h. eingesetzt worden ist. Dadurch kann vermieden werden, dass möglicherweise vor langer Zeit bereits mehrfach benutzte Lanzetten gewollt oder ungewollt wieder eingesetzt werden können.

Weiterhin kann es im Rahmen der vorliegenden Erfindung vorteilhaft sein, insbesondere im Fall einer Magazinfortschaltung vor einer Einstichbewegung, wenn das vorgesehene Magazin nur in einer vorgegeben Orientierung eingesetzt werden kann und die erste Position des Lanzettenmagazins entweder leer oder mit einem Platzhalter anstelle einer Lanzette bestückt ist. Alternativ ist es auch möglich, insbesondere bei Trommel- oder Scheibenmagazinen, die an der ersten Position befindliche Lanzette am Ende des Umlaufs mit dem Lanzettenantrieb in Kontakt zu bringen.

Andernfalls würde in diesem Fall die an erster Position des Magazins vorgesehene Lanzette nicht genutzt werden können.

Unter dem Begriff passiver Betriebszustand ist im Rahmen der vorliegenden Erfindung zu verstehen, dass die automatische Magazinfortschaltung deaktiviert ist. Dies hat zur Folge, dass vor oder nach einer Einstichbewegung einer Lanzette das Lanzettenmagazin nicht fortgeschaltet wird, so dass bei der darauf folgenden Einstichbewegung die gleiche Lanzette wie in der vorherigen Einstichbewegung erneut mit dem Verbindungselement des Lanzettenantriebs in Kontakt gebracht wird. Dies lässt sich in analoger Weise zum vorstehend beschriebenen automatischen Weitertakten beispielsweise dadurch erreichen, dass die Magazinfortschaltung mechanisch von dem Lanzettenantrieb bzw. dem Spannmechanismus entkoppelt ist und ein Spannen der Lanzettenvorrichtung bzw. das Auslösen eines Einstichvorganges nicht auch eine Fortschaltung des Lanzettenmagazins bewirkt. In diesem passiven Betriebszustand wird daher eine einmal mit dem Verbindungselement in Kontakt gebrachte Lanzette bei den darauf folgenden Einstichbewegungen so lange weiter benutzt, bis das Lanzettenmagazin auf andere Weise fortgeschaltet bzw. weitergetaktet wird.

Die erfindungsgemäße Lanzettenvorrichtung weist darüber hinaus ein Wahlelement auf, mit dem die Magazinfortschaltung wahlweise in den aktiven oder in den passiven Betriebszustand versetzt werden kann. Im Rahmen einer bevorzugten Ausführungsform ist das erfindungsgemäß vorgesehene Wahlelement so ausgebildet, dass die Magazinfortschaltung jeweils reversibel in den aktiven oder passiven Betriebszustand versetzt werden kann. Dadurch kann gewährleistet werden, dass die erfindungsgemäße Lanzettenvorrichtung wahlweise, je nach den aktuellen Bedürfnissen des Benutzers in den jeweils gewünschten Betriebszustand versetzt werden kann. So ist es beispielsweise möglich, die erfindungsgemäße Lanzettenvorrichtung mit einem vorab eingestellten Betriebszustand als Standard-Betriebszustand bereitzustellen, der dann gewünschtenfalls vom Benutzer kurzzeitig oder dauerhaft in den jeweils anderen Betriebszustand versetzt werden kann. Im Rahmen einer bevorzugten Ausführungsform wird die erfindungsgemäße Lanzettenvorrichtung so bereitgestellt, dass sich die Lanzettenfortschaltung im aktiven Betriebszustand befindet, so dass die Magazinfortschaltung das Lanzettenmagazin vor oder nach einer Einstichbewegung einer Lanzette automatisch fortschaltet, so dass bei der darauf folgenden Einstichbewegung eine noch unbenutzte Lanzette des Lanzettenmagazins mit dem Verbindungselement des Lanzettenantriebs in Kontakt gebracht wird. Dieser Betriebszustand kann durch Betätigen des erfindungsgemäß vorgesehenen Wahlelementes unterbrochen bzw. in den passiven Betriebszustand überführt werden, so dass vor oder nach einer Einstichbewegung einer Lanzette das Lanzettenmagazin nicht fortgeschaltet wird und dadurch in der darauf folgenden Einstichbewegung die gleiche Lanzette wie bei der vorherigen Einstich- und Rückführbewegung erneut mit dem Verbindungselement des Lanzettenantriebs in Kontakt gebracht wird.

Die technische Ausgestaltung des erfindungsgemäß vorgesehenen Wahlelementes unterliegt keinen grundsätzlichen Beschränkungen und kann auf vielfältige Weise in der erfindungsgemäßen Lanzettvorrichtungen realisiert sein. Bevorzugt ist das Wahlelement jedoch so ausgestaltet, dass es den Dimensionen einer üblicherweise mit einer Hand zu bedienenden Lanzettenvorrichtung Rechnung trägt und durch den Benutzer gut zugänglich ist. Im Rahmen einer bevorzugten Ausführungsform ist das Wahlelement an der Bedienoberfläche der erfindungsgemäßen Lanzettenvorrichtung angeordnet und manuell betätigbar. Dadurch kann gewährleistet werden, dass das Wahlelement von außen für den Benutzer zugänglich ist und nicht beispielsweise zunächst geöffnet oder zerlegt werden muss. Umgekehrt kann es auch vorteilhaft sein, wenn das Wahlelement so angeordnet ist, dass es nur zugänglich ist, wenn seitens des Benutzers zunächst eine weitere Aktion wie beispielsweise das Öffnen einer Abdeckung vorzunehmen ist, so dass ein unbeabsichtigtes Betätigen des Wahlelementes weitgehend ausgeschlossen werden kann. Bevorzugt ist das Wahlelement jedoch an der Bedienoberfläche angebracht und von der Außenseite der Stechhilfe zugänglich. Wiederum bevorzugt ist das Wahlelement an einer Stelle der Bedienoberfläche angeordnet, in der es bei einer Bedienung in vorgesehener Weise, bevorzugt bei einhändiger Bedienung, nicht unbeabsichtigt betätigt werden kann. Beispielsweise kann das Wahlelement auch in einer von außen zugänglichen Vertiefung der Bedienoberfläche angebracht sein. Bevorzugt ist das Wahlelement so auf der Bedienoberfläche positioniert, dass eine Benutzung der erfindungsgemäßen Stechhilfen unter gleichzeitiger Betätigung des Wahlelementes nur durch beidhändige Benutzung möglich ist.

Das erfindungsgemäß vorgesehene Wahlelement kann so ausgestaltet sein, dass die Magazinfortschaltung nach dem Betätigen des Wahlelements so lange in den gewählten Betriebszustand versetzt ist, bis durch eine weitere Betätigung des Wahlelements der jeweils andere Betriebszustand gewählt wird. Diese Ausgestaltung kann beispielsweise durch einen Schalter realisiert werden, der zwei Schalterstellungen vorsieht wie beispielsweise ein Kippschalter mit zwei Positionen des kippbaren Schaltelementes (Ein/Aus-Schalter) oder auch ein Schieber, der in zwei Positionen verschoben werden kann. Dabei ist jedoch konstruktiv sicher zu stellen, dass der jeweils gewählte Schalter nach dem Betätigen in der gewünschten Stellung verbleibt, so dass der jeweils gewünschte Betriebszustand dauerhaft bestehen bleibt.

Alternativ ist es jedoch auch möglich, dass das Wahlelement so ausgestaltet ist, dass die Magazinfortschaltung nur während der Betätigung des Wahlelementes von einem der beiden Betriebeszustände in den jeweils anderen Betriebszustand versetzt werden kann. Dies wäre beispielsweise dann gegeben, wenn das Wahlelement nach der Betätigung selbstständig, d.h. ohne weiteres Zutun des Benutzers in den Ausgangszustand zurückkehren würde. Eine mögliche technische Ausgestaltung dafür wäre beispielsweise dadurch gekennzeichnet, dass das Wahlelement als Taster mit Rückstellfeder ausgestaltet ist. Wiederum bevorzugt wäre der genannte Taster an einer Stelle der Lanzettenvorrichtung, bevorzugt an einer Stelle der Bedienoberfläche angeordnet, an der eine gleichzeitige Betätigung des Wahlelementes mit dem Bedienelement der Lanzettenvorrichtung mit einer Hand nur schwer möglich, bevorzugt nur beidhändig möglich ist.

Im Rahmen einer wiederum bevorzugten Ausführungsform wird die Lanzettenvorrichtung so bereitgestellt, dass die Magazinfortschaltung sich im voreingestellten aktiven Betriebszustand befindet. Weiterhin bevorzugt kann die Magazinfortschaltung nur während der Betätigung des Wahlelementes, beispielsweise durch Betätigung eines wie vorstehend ausgestalteten Tasters mit Rückstellfeder, in den passiven Betriebszustand versetzt werden. Es handelt sich dabei um eine besonders bevorzugte Ausgestaltung der erfindungsgemäßen Lanzettenvorrichtung, da dabei der aktive Betriebszustand nur durch ein aktives, in der Regel nicht versehentliches Eingreifen während der Benutzung deaktiviert werden kann, die Lanzettenvorrichtung jedoch selbsttätig wieder in den als Grundeinstellung vorgesehenen aktiven Betriebszustand zurückfällt. Dadurch ist gewährleistet, dass ein mehrmaliges Benutzen einer Lanzette nicht versehentlich sondern nur durch gezielte Betätigung des Wahlelementes bei jeder Benutzung der erfindungsgemäßen Lanzettenvorrichtung erfolgt.

Der passive Betriebszustand der erfindungsgemäßen Stechhilfe zeichnet sich dadurch aus, dass die automatische Magazinfortschaltung deaktiviert ist. Dabei ist der Begriff deaktiviert breit aufzufassen und kann beispielsweise bedeuten, dass der jeweils vorgesehene Mechanismus zur Magazinfortschaltung unterbrochen ist oder alternativ dazu überbrückt ist. Die beiden genanten Möglichkeiten unterscheiden sich im Wesentlichen dadurch, dass bei eine Unterbrechung der Magazinfortschaltung diese vorübergehend außer Funktion gesetzt wird, wohingegen sie bei einer Überbrückung weiterhin arbeitet, jedoch kein Fortschalten des Lanzettenmagazins mehr bewirkt. Dies kann beispielsweise dadurch bewerkstelligt werden, dass die Lanzettenfortschaltung im passiven Betriebszustand mechanisch von dem Lanzettenmagazin entkoppelt ist.

Eine weitere bevorzugte Ausführungsform der erfindungsgemäßen Lanzettenvorrichtung ist dadurch gekennzeichnet, dass die Magazinfortschaltung mit dem Lanzettenantrieb mechanisch gekoppelt ist. Dabei ist unter einer mechanischen Kopplung zu verstehen, dass im Rahmen einer Betätigung des Lanzettenantriebs, vorzugsweise im Rahmen einer Betätigung der Spanneinrichtung ein Teil der dabei aufgewendeten Kraft zum Fortschalten des Lanzettenmagazins aufgewendet wird. Beispielsweise kann bei einer Betätigung der Spanneinrichtung durch wie vorstehend beschriebenes Niederdrücken des Betätigungselementes auch die Magazinfortschaltung betätigt werden, so dass das Lanzettenmagazin fortgeschaltet wird und eine neue, noch unbenutzte Lanzette mit dem Verbindungselement des Lanzettenantriebes in Kontakt gebracht wird. Eine bevorzugte Ausführungsform der erfindungsgemäßen Lanzettenvorrichtung ist dementsprechend dadurch gekennzeichnet, dass im aktiven Betriebszustand die Magazinfortschaltung mit der Spanneinrichtung derart gekoppelt ist, dass die Fortschaltung des Lanzettenmagazins während der Betätigung der Spanneinrichtung zum Spannen der Antriebsfeder erfolgt. Das Fortschalten des Lanzettenmagazins erfolgt dann wie vorstehend beschrieben unmittelbar vor der Einstichbewegung.

Alternativ kann das Fortschalten des Lanzettenmagazins auch so mit dem Lanzettenantrieb bzw. der Spanneinrichtung gekoppelt sein, dass die nach einer Einstich- und Rückführbewegung einer Lanzette noch verbleibenden Restenergie des Lanzettenantriebs auf die Magazinfortschalung übertragen wird, so dass im unmittelbaren Anschluss an eine Einstichbewegung, gegebenenfalls noch während der Rückführbewegung der Lanzette, das Lanzettenmagazin fortgeschaltet und so eine neue Lanzette zur Verfügung gestellt wird.

Bevorzugt ist die erfindungsgemäße Lanzettenvorrichtung allerdings so ausgestaltet, dass lediglich im aktiven Betriebszustand die Magazinfortschaltung mit dem Lanzettenantrieb mechanisch gekoppelt ist. Im passiven Betriebszustand ist die Magazinfortschaltung dementsprechend bevorzugt mechanisch von dem Lanzettenantrieb entkoppelt, wobei die Entkopplung wie vorstehend ausgeführt bevorzugt reversibel, d.h. umkehrbar ist und besonders bevorzugt nur während der Betätigung des Wahlelementes andauert oder solange andauert, bis die Lanzettenfortschaltung durch Betätigung des Wahlelementes in den aktiven Betriebszustand zurückversetzt wird. Dabei ist, wie vorstehend ausgeführt, die Entkopplung bevorzugt so ausgeführt, dass die Magazinfortschaltung lediglich überbrückt wird, d.h. weiterhin arbeitet, wobei jedoch kein Fortschalten des Lanzettenmagazins mehr bewirkt wird.

Eine solche mechanische Kopplung lässt sich beispielsweise durch ein sogenanntes Schrittschaltwerk realisieren. Im Rahmen einer bevorzugten Ausführungsform betrifft die vorliegende Erfindung daher eine Lanzettenvorrichtung, die dadurch gekennzeichnet ist, dass die erfindungsgemäß vorgesehene Lanzettenfortschaltung ein Schrittschaltwerk umfasst. Schrittschaltwerke an sich sind dem Fachmann bekannt und finden beispielsweise bei der Konstruktion von Filmprojektoren, bei denen der Film weitergetaktet werden muss, oder auch im Uhrenbau Anwendung. Grundsätzlich können jedoch auch andere Translations- oder Rotationsbewegungen von Gegenständen unter Einsatz eines Schrittschaltwerkes getaktet werden. Ein Schrittschaltwerk zeichnet sich in der Regel dadurch aus, dass es einen Greifmechanismus aufweist, der eine periodische, sich immer wiederholende und bei jeder Wiederholung gleichförmige Bewegung ausführt. Dieser Greifmechanismus wird üblicherweise mit einer Gegenstruktur bzw. einem Gegenelement des zu bewegenden bzw. zu rotierenden Körpers in mechanischen Eingriff gebracht. Dabei wird die periodische Bewegung des Greifelementes in eine getaktete Bewegung, vorzugsweise Rotation des zu bewegenden Körpers übertragen.

Dementsprechend ist eine weitere bevorzugte Ausführungsform der erfindungsgemäßen Lanzettenvorrichtung dadurch gekennzeichnet, dass das Schrittschaltwerk einen beweglichen Greifer sowie mehrere Gegenelemente umfasst, wobei der bewegliche Greifer so angeordnet ist, dass er mit den Gegenelementen in Eingriff gebracht werden kann, wodurch das Lanzettenmagazin fortgeschaltet wird. Eine vorteilhafte Ausgestaltung einer derartigen Vorrichtung ist dadurch gekennzeichnet, dass der bewegliche Greifer des Schrittschaltwerkes Bestandteil der Lanzettenvorrichtung ist. Im Rahmen dieser vorteilhaften Ausgestaltung ist es wiederum bevorzugt, wenn die zum Eingriff mit dem vorrichtungsseitigen beweglichen Greifer vorgesehenen Gegenelemente Bestandteile des Lanzettenmagazins sind. Der Erfindung betrifft daher im Rahmen dieser Ausführungsform auch eine wie vorstehend beschriebene Lanzettenvorrichtung, die auch ein Lanzettenmagazin umfasst, wobei das Lanzettenmagazin vorzugsweise Gegenelemente aufweist, die so angeordnet sind, dass sie mit dem beweglichen Greifer eines vorrichtungsseitig vorgesehenen Schrittschaltwerkes in Eingriff gebracht werden können, wodurch das Lanzettenmagazin fortgeschaltet werden kann.

Daneben ist es im Rahmen einer alternativen Ausführungsform auch möglich, dass Gegenelemente des Schrittschaltwerkes nicht unmittelbar auf dem fortzuschaltenden Lanzettenmagazin angeordnet sind sondern auf einem beweglichen Magazinträger, der sich vorzugsweise in der Magazinaufnahme der Lanzettenvorrichtung befindet. Der im Rahmen dieser Ausführungsform vorgesehene Magazinträger ist vorzugsweise so ausgestaltet, dass er mechanisch fest aber lösbar mit einem wie vorstehend beschriebenen Lanzettenmagazin verbunden werden kann. Ein Fortschalten das Magazins, beispielsweise durch Verschieben oder bevorzugt, im Falle eines rotationssymmetrischen Lanzettenmagazins, durch Drehen kann dann auch durch Verschieben bzw. Drehen des ebenfalls vorrichtungsseitig angebrachten Magazinträgers bewerkstelligt werden. In diesem Fall ist es auch von Vorteil, wenn der bewegliche Magazinträger die Gegenelemente des Schrittschaltwerkes aufweist, die mit dem beweglichen Greifer in Eingriff gebracht werden sollen. Eine bevorzugte Ausführungsform der erfindungsgemäßen Lanzettenvorrichtung ist demnach dadurch gekennzeichnet, dass die Magazinaufnahme einen beweglichen Magazinträger umfasst, wobei die Gegenelemente des Schrittschaltwerkes Bestandteil des beweglichen Magazinträgers sind. Im Hinblick auf eine reduzierte Baugröße, die bei Lanzettenvorrichtungen des vorliegenden Typs wünschenswert ist, sowie im Hinblick auf eine reduzierte Anteil von Bauteilen ist es jedoch vorteilhaft, die Gegenelemente des Schrittschaltwerkes wie vorstehend beschrieben auf dem Lanzettenmagazin vorzusehen und den vorrichtungsseitigen beweglichen Greifer so anzuordnen, dass er mit diesen direkt in Eingriff gebracht werden kann.

Durch das erfindungsgemäß bevorzugt vorgesehene Schrittschaltwerk lässt sich in vorteilhafter Weise auch bewerkstelligen, dass die Magazinfortschaltung wie vorstehend beschrieben im passiven Betriebszustand lediglich überbrückt wird. Dies kann beispielsweise dadurch erreicht werden, dass im passiven Betriebszustand der Magazinfortschaltung der bewegliche Greifer des Schrittschaltwerks nicht in Eingriff mit den Gegenelementen kommt. Beispielsweise kann der bewegliche Greifer durch Einschalten des passiven Betriebszustandes so verlagert werden, dass er zwar seine wiederkehrende gleichförmige Greifbewegung weiterhin ausführt, jedoch nicht mehr in Eingriff mit den Gegenelementen des Schrittschaltwerkes gelangt. Dies kann beispielsweise durch eine vorübergehende, reversible Verdrehung oder Verschiebung des Schrittschaltwerkes, vorzugsweise nur des beweglichen Greifers erfolgen. Beispieles weise könnte durch oder während einer Betätigung des Wahlelementes der bewegliche Greifer des Schrittschaltwerkes so verlagert werden, dass er nicht mehr mit den Gegenelementen in Eingriff kommen würde, wodurch die Fortschaltung des Lanzettenmagazins während der Betätigung oder, je nach Ausgestaltung, bis zur erneuten Betätigung des Wahlelementes unterbrochen würde. Dadurch könnte gewährleistet werden, dass eine automatisch vorgesehene Magazinfortschaltung nur willentliche und gezielt unterbrochen werden könnte. Dies würde dem Anwender einer erfindungsgemäßen Stechhilfe bei maximaler Sicherheit durch ein automatisches Weitertakten ein Höchstmaß an Flexibilität eröffnen und jederzeit die möglicherweise wünschenswerte Wiederbenutzung einer bereits gebrauchten Lanzette ermöglichen.

Die vorliegende Erfindung betrifft in einem weiteren Aspekt ein Entnahmesystem zur Entnahme einer Probe einer Körperflüssigkeit umfassend eine wie vorstehend beschriebene Lanzettenvorrichtung und ein dazu passendes Lanzettenmagazin mit mehreren Lanzetten, wobei die jeweils beschriebenen bevorzugten Ausführungsformen auch bevorzugte Merkmale des erfindungsgemäßen Entnahmesystems darstellen. Bevorzugt handelt es sich dementsprechend bei dem Lanzettenmagazin dabei um ein rotationssymmetrische Magazin, insbesondere um ein wie vorstehend beschriebenes trommelförmiges Magazin. Der Lanzettenantrieb umfasst auch im Rahmen dieses Aspektes der vorliegenden Erfindung vorzugsweise ein Schrittschaltwerk. Auch im Rahmen dieses Aspektes der vorliegenden Erfindung ist es bevorzugt, dass die Gegenelemente eines vorrichtungsseitig vorgesehenen Schrittschaltwerkes auf dem Lanzettenmagazin angebracht sind.

Die erfindungsgemäße Lanzettenvorrichtung eröffnet eine Möglichkeit Notsituationen vorzubeugen in der ein Anwender eine Blutprobe, beispielsweise zur Messung des Blutglukosespiegels durchführen muss, jedoch bei aufgebrauchtem Lanzettenvorrat kein Reservemagazin zur Hand hat In diesem Fall würde bei einem aktiven automatischen Magazintransport die weitere Nutzung der Stechhilfe solange nicht möglich sein bis ein neues Magazin eingesetzt würde. Durch die erfindungsgemäß vorgesehene Möglichkeit zur Überbrückung des Fortschaltmechanismus kann jedoch die letzte Lanzette wiederverwendet werden und somit die weitere Nutzung der Stechhilfe kurzfristig sichergestellt werden.

Eine darüber hinaus gehende Verbesserung der Bedienungssicherheit und Hygiene könnte erreicht werden, wenn das erfindungsgemäße System mit einem Wiedereinlege- bzw. Wiederverwendungsschutz für benutzte und bereits entnommene Magazine gekoppelt würde. Ein solcher Wiedereinlegeschutz für Lanzettenmagazine wird bspw. in US 2004/0260325 A1 (Kuhr et al) beschrieben.

Schließlich bietet die erfindungsgemäße Lanzettenvorrichtung weitestgehende Flexibilität im Hinblick auf individuelle Nutzergewohnheiten die oft regional geprägt sind. Beispielsweise ermöglicht die erfindungsgemäße Lanzettenvorrichtung eine variable Voreinstellung der Stechhilfe durch den Hersteller im aktiven oder passiven Betriebszustand wodurch auf spezifische Nutzergewohnheiten eingegangen werden kann. In diesem Zusammenhang ist es insbesondere vorteilhaft, wenn das erfindungsgemäß vorgesehene Wahlelement verdeckt angebracht oder mit weiteren Sicherungselementen versehen ist.

Weitere Einzelheiten und Vorteile der Erfindung werden im Folgenden anhand eines Ausführungsbeispieles näher erläutert. Gleiche Bezugszeichen bezeichnen dabei gleiche bzw. funktionsgleiche oder hinsichtlich ihrer Funktion einander entsprechende Elemente. Die Funktionsweise des erfindungsgemäß bevorzugt vorgesehenen Schrittschaltwerkes ist anhand eines scheibenförmigen Lanzettenmagazins näher erläutert, ist jedoch nicht darauf beschränkt und kann genauso mit anderen Magazinformen wie beispielsweise Trommelmagazinen, Flachmagazinen Stapelmagazinen Bandmagazinen, Gurtmagazinen oder weiteren Magazinformen realisiert werden. Die dargestellten Merkmale können einzeln oder in Kombination verwendet werden, um bevorzugte Ausführungsformen zu schaffen. Es zeigen:
- Fig. 1: eine erfindungsgemäße Lanzettenvorrichtung mit Wahlelement in einer perspektivischen Seitenansicht,
- Fig. 2: eine perspektivische Ansicht eines erfindungsgemäß einsetzbaren Schrittschaltwerkes mit Lanzettenmagazin,
- Fig. 3A bis E: Schrittschaltwerk aus Fig. 2 im aktiven Betriebszustand (beweglicher Greifer im Eingriff mit Gegenelementen)
- Fig. 4A bis E: Schrittschaltwerk aus den Fig. 2 und 3 im passiven Betriebszustand (beweglicher Greifer außer Eingriff)

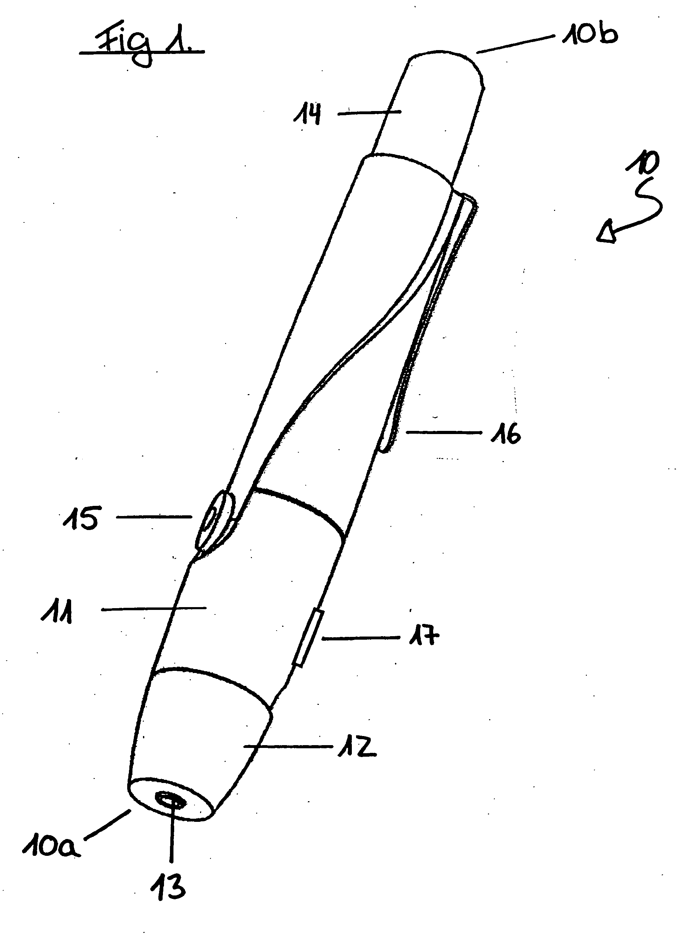

Figur 1 zeigt beispielhaft eine im Rahmen der vorliegenden Erfindung einsetzbare Lanzettenvorrichtung (10) mit Wahlelement (17) in einer perspektivischen Seitenansicht. Die beispielhaft dargestellt Lanzettenvorrichtung (10) weist ein proximales Ende (10a) und ein distales Ende (10b) sowie ein etwa spindelförmiges bzw. bezüglich einer Gerätehauptachse näherungsweise rotationssymmetrisches Gehäuse (11) auf. Das Gehäuse (11) wiederum weist eine nach außen gewandte bzw. von außen durch einen Benutzer zugängliche Bedienoberfläche sowie einen Befestigungsclip (16) zur Befestigung der Lanzettenvorrichtung auf, mit dem sie beispielsweise an der Kleidung des Benutzers bei Nichtgebrauch befestigt werden kann. Die genannte Gerätehauptachse verläuft durch die proximale, bei bestimmungsgemäßem Gebrauch dem zu stechenden Körperteil zugewandten Kontaktfläche der Stechhilfe (10). Am genannten proximalen Ende (10a) der Stechhilfe (10) ist im Rahmen des vorliegenden Beispiels eine abnehmbare Kappe (12) angebracht, die üblicherweise einen nicht dargestellten Hohlraum verschließt, der zur Aufnahme eines Lanzettenmagazins geeignet ist. Die weist wie in Figur 1 dargestellt wiederum an ihrem proximalen Ende, d.h. an deren Kontaktfläche eine Austrittöffnung (13) auf, durch die eine in Einstichrichtung bewegte Lanzette aus dem Gehäuse der Lanzettenvorrichtung austreten kann, um eine Einstichwunde in dem zu stechenden Körperteil zu erzeugen. Die genannte Austrittsöffnung kann dabei je nach Ausgestaltung des vorgesehenen Lanzettenmagazins sowohl zentral, (wie in Figur 1 dargestellt) als auch dezentral auf der Kontaktfläche der Kappe (12) angeordnet sein.

Die in Figur 1 dargestellte Stechhilfe (10) weist darüber hinaus ein Wahlelement (17) auf, das im vorliegenden Beispiel an der Außenseite, d.h. der Bedienoberfläche des Gehäuses (11) angebracht ist. Es kann in der in Figur 1 dargestellten Form sowohl als Schieber ausgebildet sein, der sich in zwei Positionen bewegen lässt, wodurch einer der beiden vorstehend beschriebenen Betriebszustände entweder dauerhaft oder temporär gewählt werden kann. Alternativ ist es auch möglich, das Wahlelement (17) in Form eines Druckschalters oder Tasters auszugestalten. Insbesondere in der Ausgestaltung als Taster eignet er sich dann zur temporären Aktivierung eines Betriebszustandes, der beispielsweise so lange vorhält, wie der das als Taster ausgestaltete Wahlelement (17) betätigt ist. Alternativ kann das Wahlelement (17) wie vorstehend beschrieben auch als Kippschalter oder in anderen geeigneten Formen ausgestaltet sein. Das in Figur 1 beispielhaft dargestellt Wahlelement (17) ist an der Bedienoberfläche des Gehäuses (11) angebracht und in dieser Form von außen für den Benutzer zugänglich. Es ist allerdings auch möglich, das Wahlelement (17) wie vorstehend beschrieben in einer verdeckten Position oder auch auf der Innenseite der erfindungsgemäßen Lanzettenvorrichtung anzubringen. In der in Figur 1 beispielhaft dargestellten Form befindet sich das Wahlelement in einer Position, in der es nicht versehentlich gleichzeitig mit anderen Bedienelementen der erfindungsgemäßen Lanzettenvorrichtung (10) betätigt werden kann. Dies gilt umso mehr bei der bevorzugten einhändigen Bedienung der erfindungsgemäßen Lanzettenvorrichtung.

Die in Figur 1 beispielhaft dargestellte Lanzettenvorrichtung (10) weist darüber hinaus ein als Taster oder Druckschalter ausgestaltetes Auslöseelement (15) auf. Durch Betätigen des Auslöseelements (15) kann die Einstichbewegung der jeweils gewählten Lanzette ausgelöst werden. Darüber hinaus weist die dargestellte Lanzettenvorrichtung ein am distalen Ende (10b) der Lanzettenvorrichtung (10) aus dem Gehäuse (11) herausragendes Bedienelement (14) auf. Durch Betätigung des Bedienelementes (14) kann der vorstehend beschriebene, in Figur 1 nicht dargestellte Lanzettenantrieb gespannt werden. Das Spannen des Lanzettenantriebs kann beispielsweise durch eine Drehbewegung des Bedienelementes um die Hauptdrehachse der Lanzettenvorrichtung (10) oder beispielsweise auch durch Verschieben des Bedienelementes (14) in Richtung des proximalen Endes (10a) der Lanzettenvorrichtung bewerkstelligt werden.

Die genannten Bedienelemente der in Figur 1 beispielhaft dargestellten Lanzettenvorrichtung (10) sind vorteilhaft an räumlich voneinander entfernten Positionen der Bedienoberfläche lokalisiert. In der dargestellten Form ist das Wahlelement (17) zwar auf gleicher Höhe des Auslöseelementes (15), jedoch in dazu diametral entgegen gesetzter Position angeordnet, wodurch ein gleichzeitige Betätigung bei Bedienung mit einer Hand weitgehend ausgeschlossen ist. Diese Anordnung wäre insbesondere für eine Lanzettenvorrichtung mit Magazinfortschaltung unmittelbar vor einer Einstichbewegung, d.h. während des Spannens des Lanzettenantriebs, vorteilhaft. Dieser Effekt ließe sich beispielsweise noch dadurch unterstützen, dass das Wahlelement (17) auf einer vertieften Position der Bedienoberfläche angebracht wäre.

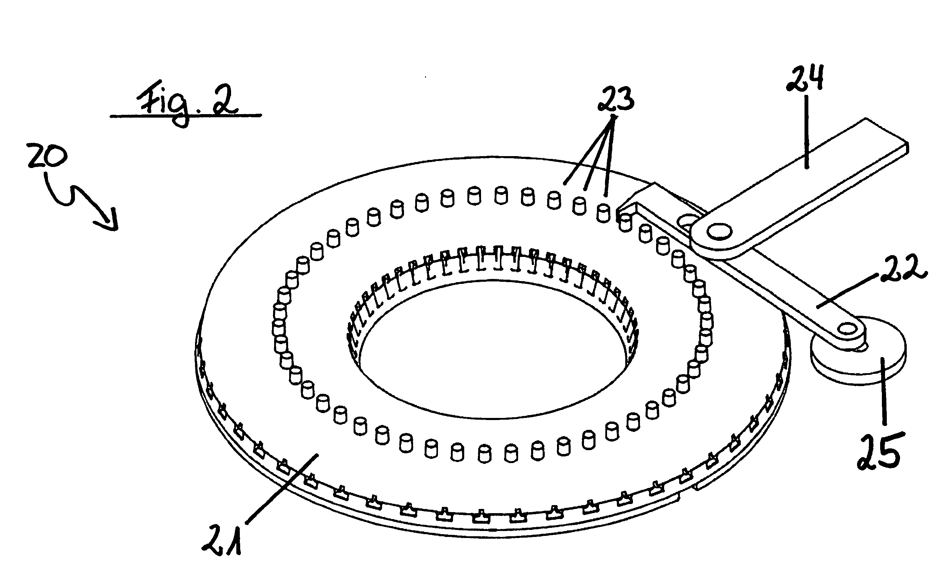

In Figur 2 ist beispielhaft ein im Rahmen einer bevorzugten Ausführungsform der erfindungsgemäßen Lanzettenvorrichtung (10) vorgesehenes Schrittschaltwerk (20) dargestellt. In der dargestellten Form ist der zur bewegende bzw. zu rotierende Körper als kreisscheibenförmiges Lanzettenmagazin (21) ausgebildet. Das Lanzettenmagazin (21) trägt auf seiner Oberseite eine Mehrzahl von Gegenelementen (23), die kreisförmig und konzentrisch zum Magazin (21) angeordnet sind. Alternativ ist es allerdings auch möglich, dass die Gegenelemente (23) des Schrittschaltwerkes wie vorstehend beschrieben nicht unmittelbar auf dem Lanzettenmagazin (21) angebracht sind, sondern auf einem Magazinträger, der mechanisch fest aber lösbar mit einem Lanzettenmagazin verbunden werden kann.

Das in Figur 2 dargestellte Schrittschaltwerk weist darüber hinaus einen beweglichen Greifer (22) auf. Dieser kann durch eine stets gleichförmige Bewegung mit den auf dem Magazin (21) angebrachten Gegenelementen (23) in mechanischen Eingriff gebracht werden. Dadurch kann die periodisch translatorische Bewegung des Greifers in eine getaktete Drehung des wie in Figur 2 dargestellt als Scheibenmagazin (21) ausgebildeten zu drehenden Körpers übertragen werden. Die stets gleichförmige Hin- und Herbewegung des Greifers (23) kann beispielsweise durch einen geeigneten rotierenden Antrieb (25) bewerkstelligt werden.

Das in Figur 2 beispielhaft dargestellte Schrittschaltwerk (20) weist darüber hinaus ein mit dem beweglichen Greifer (22) mechanisch verbundenes Steuerelement (24) auf. Das dargestellte Steuerelement (24) ist so angeordnet, dass es den bewegliche Greifer (22) bezüglich seiner räumlichen Anordnung vorübergehend so verlagern kann, dass dieser nicht mehr mit den Gegenelementen (23) des zu drehenden Körpers in Eingriff gebracht werden kann. Dadurch kann gewährleistet werden, dass trotz Fortsetzung der gleichförmigen Bewegung des Greifers (22) der zu drehende Körper, hier das dargestellte Lanzettenmagazin (21) nicht mehr weitergedreht wird wie es im passiven Betriebszustand der erfindungsgemäßen Lanzettenvorrichtung vorgesehen ist. Aus diesem passiven Betriebszustand, in dem trotz fortgesetzter Bewegung des Greifers (23), beispielsweise nach Betätigen des Betätigungselementes (14), keine Drehung, d.h. kein Fortschalten des Lanzettenmagazins (21) mehr stattfindet, kann durch Verlagerung des Steuerelementes (24) in den Ausgangszustand wieder der aktive Betriebszustand hergestellt werden. In diesem aktiven Betriebszustand befindet sich der bewegliche Greifer (22) in einer Position, in der er bei seiner gleichförmigen wiederkehrenden Bewegung mit den Gegenelementen (23) des Schrittschaltwerkes (20) in Eingriff kommt.

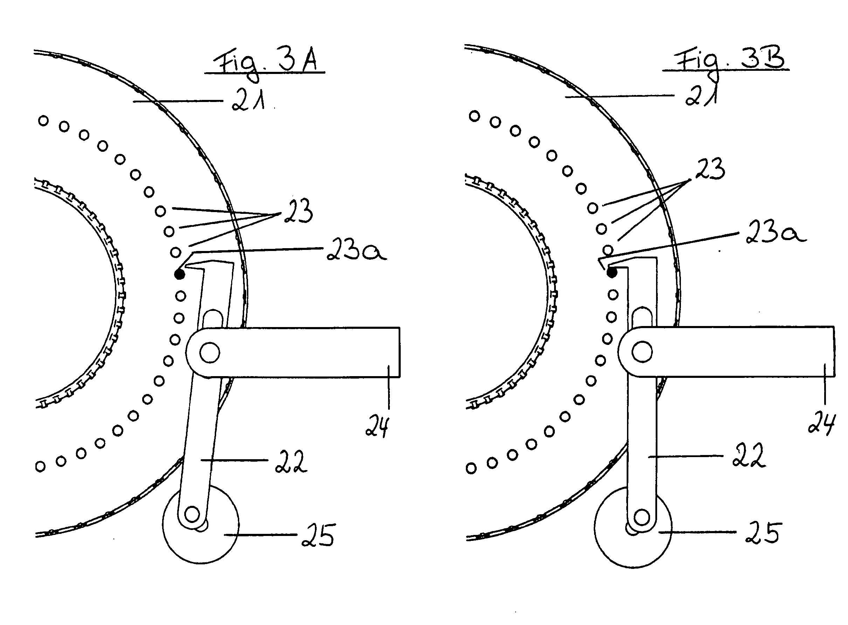

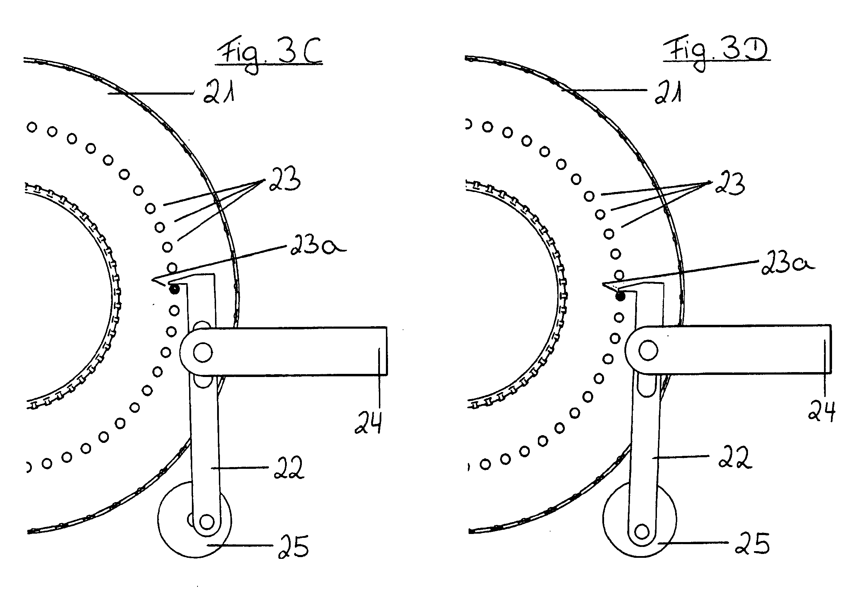

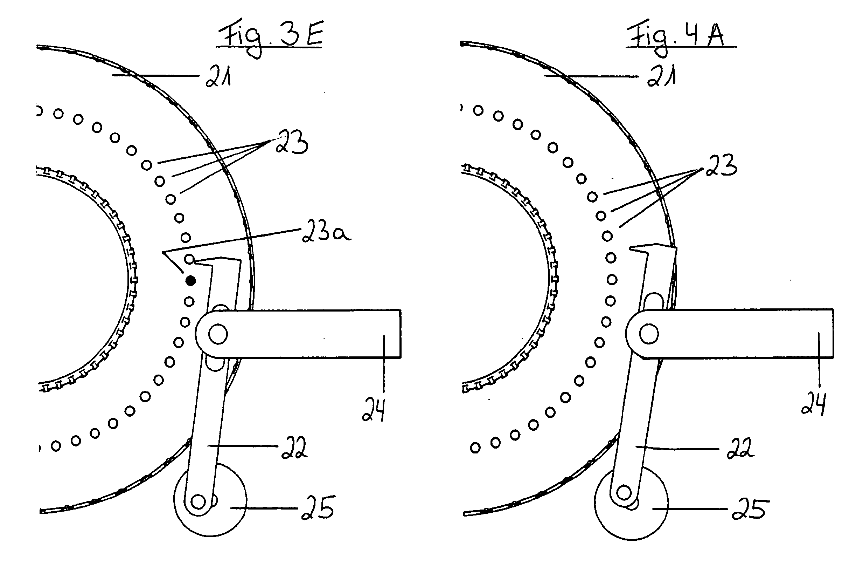

Der Bewegungsablauf des beispielhaft dargestellten Schrittschaltwerkes (20) im aktiven Betriebszustand ist in den Figuren 3A bis 3E dargestellt. Figur 3A stellt den Ausgangspunkt des Bewegungsablaufes dar. Dabei ist der Greifer (22) durch das Steuerelement (24) so in Richtung des Scheibenmagazins (21) verschoben, dass er mit den Gegenelementen (23) in Eingriff kommen kann. Diese Situation ist in Figur 3B dargestellt, wobei sich der Greifer (22) durch Drehung des Antriebs (25) im Uhrzeigersinn in Richtung der Gegenelemente (23) des Schrittschaltwerkes geneigt hat und mit dem zur Veranschaulichung markierten Gegenelement (23a) in Kontakt kommt. Wie in Figur 3C dargestellt führt eine weitere Drehung des Antriebs (25) dazu, dass sich der Greifer in Richtung des unteren Bildrandes bewegt und dabei das mit dem Greifer (22) in Kontakt befindliche Gegenelement (23a) mitnimmt. Dies führt zu einer Drehung des Scheibenmagazins (21) im Uhrzeigersinn bis zu der in Figur 3D dargestellten Situation, in der sich die Bewegungsrichtung des Greifers (22) umkehrt, wodurch anschließend der Kontakt mit dem Gegenelement (23a) aufgehoben wird. Dies ist in der abschließenden Figur 3E dargestellt, in der der Greifer (22) vor der Herstellung des Kontaktes mit dem nächsten Gegenelement ist, bzw., je nach Konstruktion, bevor er mit einem der nächsten Gegenelemente (23) in Kontakt kommt. Dadurch kann bewerkstelligt werden, dass die periodisch wiederkehrende Bewegung des Greifers in eine gleichförmig getaktete Rotation, d.h. in ein Fortschalten des Lanzettenmagazins überführt wird.

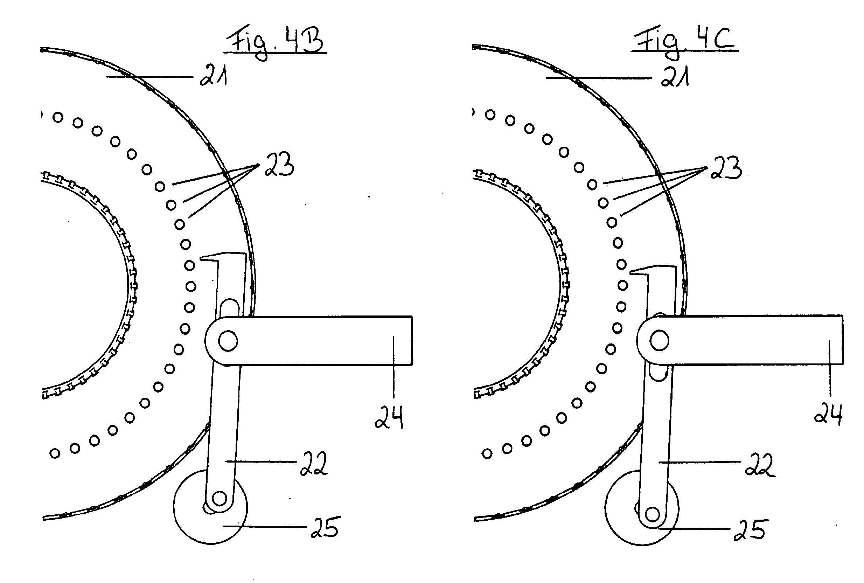

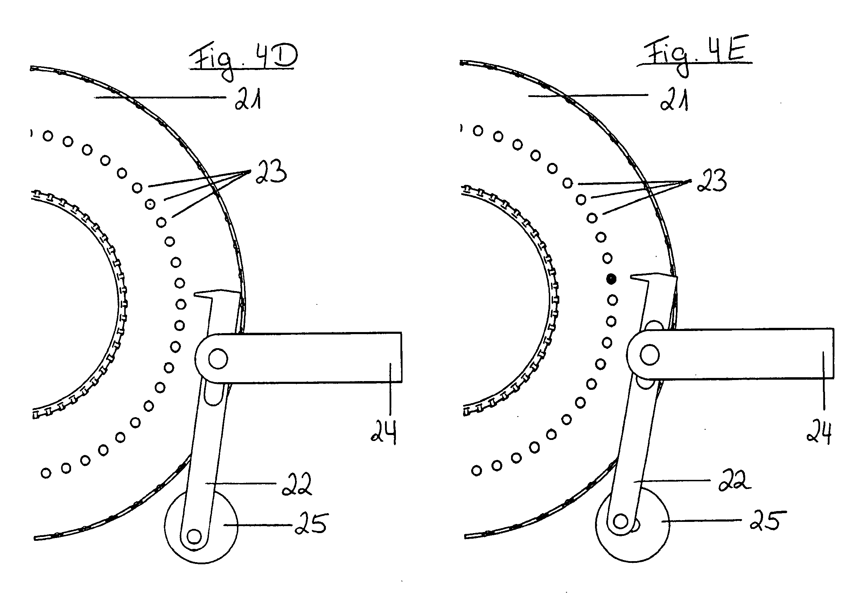

Die Figuren 4A bis 4E hingegen zeigen den analogen Bewegungsablauf des Schrittschaltwerkes (20) im passiven Betriebszustand. Dabei ist die Position des Greifers (22) durch ein verschieben des Steuerelementes (24) vom Scheibenmagazin (21) weg so verlagert, dass der Greifer (22) über den gesamten wiederkehrenden Bewegungsablauf hinweg nicht mit einem der Gegenelemente (23) in Kontakt kommt. Dadurch führt selbst die fortgesetzte Bewegung des Greifers (22) nicht zu einer Rotation bzw. einem Fortschalten des Lanzettenmagazins.

### Bezugszeichenliste:

- 10: Lanzettenvorrichtung mit proximalem Ende (10a) und distalem Ende (10b)
- 11: Gehäuse mit Bedienoberfläche
- 12: Kappe
- 13: Austrittsöffnung des Gehäuses
- 14: Betätigungselement (Spannknopf)
- 15: Auslöseelement
- 16: Befestigungsclip
- 17: Wahlelement
- 20: Schrittschaltwerk
- 21: Lanzettenmagazin
- 22: Beweglicher Greifer
- 23, 23a: Gegenelemente
- 24: Steuerelement
- 25: Antrieb

## Patentansprüche

1. Lanzettenvorrichtung (10) zum Erzeugen einer Einstichwunde, umfassend
- eine Magazinaufhahme für ein Lanzettenmagazin (21) mit mehreren Lanzetten; **gekennzeichnet durch**
- einen Lanzettenantrieb mit einer Antriebsfeder, einer Spanneinrichtung zum Spannen der Antriebsfeder und einem Verbindungselement, das mit einer in dem Lanzettenmagazin (21) angeordneten Lanzette in Kontakt gebracht werden kann und das mittels des Lanzettenantriebs die mit ihm in Kontakt gebrachte Lanzette zu einer Einstichbewegung bewegen kann um eine Einstichwunde zu erzeugen;
- eine Magazinfortschaltung, mit der das Lanzettenmagazin (21) so fortschaltbar ist, dass die in ihm gelagerten Lanzetten nacheinander mit dem Verbindungselement in Kontakt gebracht werden können und die einen aktiven und einen passiven Betriebszustand aufweist und
- ein Wahlelement (17), mit dem die Magazinfortschaltung wahlweise in den aktiven oder in den passiven Betriebszustand versetzt werden kann,
wobei im aktiven Betriebszustand die Magazinfortschaltung das Lanzetterumagazin (21) vor oder nach einer Einstichbewegung einer Lanzette automatisch fortschaltet, so dass bei der darauf folgenden Einstichbewegung eine noch unbenutzte Lanzette des Lanzettenmagazins (21) mit dem Verbindungselement des Lanzettenantriebs in Kontakt gebracht wird und wobei im passiven Betriebszustand die automatische Magazinfortschaltung deaktiviert ist, so dass vor oder nach einer Einstichbewegung einer Lanzette das Lanzettenmagazin (21) nicht fortgeschaltet wird, so dass bei der darauf folgenden Einstichbewegung die gleiche Lanzette wie bei der vorherigen Einstichbewegung erneut mit dem Verbindungselement des Lanzettenantriebs in Kontakt gebracht wird.

2. Lanzettenvorrichtung nach Anspruch 1, **dadurch gekennzeichnet, dass** das Verbindungselement mit einer in dem Lanzettenmagazin (21) angeordneten Lanzette derart in Kontakt gebracht werden kann, dass es an die in dem Lanzettenmagazin (21) angeordnete Lanzette ankuppelbar ist und mittels des Lanzettenantriebs zusammen mit der daran angekuppelten Lanzette zu einer Einstichbewegung bewegt werden kann um eine Einstichwunde zu erzeugen.

3. Lanzettenvorrichtung nach Anspruch 1 oder 2, **dadurch gekennzeichnet, dass** das Verbindungselement als Schubstange ausgebildet ist.

4. Lanzettenvorrichtung nach einem der vorstehenden Ansprüche, **dadurch gekennzeichnet, dass** das Wahlelement (17) so ausgebildet ist, dass die Magazinfortschaltung jeweils reversibel in den aktiven oder passiven Betriebszustand versetzt werden kann.

5. Lanzettenvorrichtung nach einem der vorstehenden Ansprüche, **dadurch gekennzeichnet, dass** das Wahlelement (17) so ausgestaltet ist, dass die Magazinfortschaltung nach dem Betätigen des Wahlelements (17) so lange in den gewählten Betriebszustand versetzt ist, bis durch eine weitere Betätigung des Wahlelements der jeweils andere Betriebszustand gewählt wird.

6. Lanzettenvorrichtung nach einem der vorstehenden Ansprüche, **dadurch gekennzeichnet, dass** das Wahlelement (17) so ausgestaltet ist, dass die Magazinfortschaltung nur während der Betätigung des Wahlelementes (17) von einem der beiden Betriebeszustände in den jeweils anderen Betriebszustand versetzt werden kann.

7. Lanzetten Vorrichtung nach einem der vorstehenden Ansprüche, **dadurch gekennzeichnet, dass** im aktiven Betriebszustand die Magazinfortschaltung mit dem Lanzettenantrieb (25) mechanisch gekoppelt ist.

8. Lanzettenvorrichtung nach einem der vorstehenden Ansprüche, **dadurch gekennzeichnet, dass** im aktiven Betriebszustand die Magazinfortschaltung mit der Spanneinrichtung derart gekoppelt ist, dass die Fortschaltung des Lanzettenmagazins (21) während der Betätigung der Spanneinrichtung zum Spannen der Antriebsfeder erfolgt.

9. Lanzettenvorrichtung nach einem der vorstehenden Ansprüche, **dadurch gekennzeichnet, dass** die Lanzettenfortschaltung ein Schrittschaltwerk (20) umfasst.

10. Lanzettenvorrichtung nach Anspruch 9, **dadurch gekennzeichnet, dass** das Schrittschaltwerk (20) einen beweglichen Greifer (22) sowie mehrere Gegenelemente (23, 23a) umfasst, wobei der bewegliche Greifer (22) so angeordnet ist, dass er mit den Gegenelementen (23, 23a) in Eingriff gebracht werden kann, wodurch das Lanzettenmagazin (21) fortgeschaltet wird.

11. Lanzettenvorrichtung nach Anspruch 9 oder 10, **dadurch gekennzeichnet, dass** der bewegliche Greifer (22) des Schrittschaltwerkes (20) Bestandteil der Lanzettenvorrichtung (10) ist.

12. Lanzettenvorrichtung nach einem der Ansprüche 10 oder 11, **dadurch gekennzeichnet, dass** die Gegenelemente (23, 23a) Bestandteil des Lanzettenmagazins (21) sind.

13. Lanzettenvorrichtung nach einem der Ansprüche 10 bis 12, **dadurch gekennzeichnet, dass** die Magazinaufnahme einen beweglichen Magazinträger umfasst, wobei die Gegenelemente (23, 23a) Bestandteil des beweglichen Magazinträgers sind.

14. Lanzettenvorrichtung nach einem der Ansprüche 10 bis 13, **dadurch gekennzeichnet, dass** im passiven Betriebszustand der Magazinfortschaltung der bewegliche Greifer (22) des Schrittschaltwerks nicht in Eingriff mit den Gegenelementen (23, 23a) kommt.

15. Entnahmesystem zur Entnahme einer Probe einer Körperflüssigkeit umfassend eine Lanzettenvorrichtung (10) nach einem der vorstehenden Ansprüche und ein dazu passendes Lanzettenmagazin (21) mit mehreren Lanzetten.

## Claims

1. Lancet device (10) for producing a puncture wound comprising
- a magazine holder for a lancet magazine (21) with several lancets; **characterized by**
- a lancet drive with a drive spring, a tensioning device for tensioning the drive spring and a connecting element which can be brought into contact with a lancet arranged in the lancet magazine (21) and which, by means of the lancet drive, can move the lancet that has been brought into contact with it for a puncturing movement in order to produce a puncture wound;
- a magazine advancing mechanism which allows the lancet magazine (21) to be advanced in such a manner that the lancets stored therein can be successively brought into contact with the connecting element and which has an active and a passive operating state and
- a selection element (17) with which the magazine advancing mechanism can be selectively shifted into the active or into the passive operating state,
wherein in the active operating state the magazine advancing mechanism automatically advances the lancet magazine (21) before or after a puncturing movement of a lancet such that an as yet unused lancet of the lancet magazine (21) is brought into contact with the connecting element of the lancet drive in the subsequent puncturing movement and wherein in the passive operating state the automatic magazine advancing mechanism is deactivated such that the lancet magazine (21) is not advanced before or after a puncturing movement of a lancet so that in the subsequent puncturing movement the same lancet as in the previous puncturing movement is again brought into contact with the connecting element of the lancet drive.

2. Lancet device according to claim 1, **characterized in that** the connecting element can be brought into contact with a lancet located in the lancet magazine (21) in such a manner that it can be coupled to the lancet located in the lancet magazine (21) and can be moved together with the lancet coupled thereto to make a puncturing movement by means of the lancet drive in order to produce a puncture wound.

3. Lancet device according to claim 1 or 2, **characterized in that** the connecting element is designed as a push rod.

4. Lancet device according to one of the previous claims, **characterized in that** the selection element (17) is designed such that the magazine advancing mechanism can in each case be reversibly shifted into the active or passive operating state.

5. Lancet device according to one of the previous claims, **characterized in that** the selection element (17) is designed such that the magazine advancing mechanism is shifted into the selected operating state after actuating the selection element until the other respective operating state is selected by further actuation of the selection element.

6. Lancet device according to one of the previous claims, **characterized in that** the selection element (17) is designed such that the magazine advancing mechanism can only be shifted from one of the two operating states into the other respective operating state during actuation of the selection element.

7. Lancet device according to one of the previous claims, **characterized in that** the magazine advancing mechanism is mechanically coupled to the lancet drive (25) in the active operating state.

8. Lancet device according to one of the previous claims, **characterized in that** in the active operating state the magazine advancing mechanism is coupled to the tensioning device in such a manner that the lancet magazine (21) is advanced during actuation of the tensioning device to tension the drive spring.

9. Lancet device according to one of the previous claims, **characterized in that** the lancet advancing mechanism comprises a step-switching mechanism (20).

10. Lancet device according to claim 9, **characterized in that** the step-switching mechanism (20) comprises a movable gripper (22) as well as several counter elements (23, 23a), where the movable gripper (22) is arranged such that it can be engaged with the counter elements (23, 23a) resulting in an advance of the lancet magazine (21).

11. Lancet device according to claim 9 or 10, **characterized in that** the movable gripper (22) of the step-switching mechanism (20) is a component of the lancet device (10).

12. Lancet device according to one of the claims 10 or 11, **characterized in that** the counter elements (23, 23a) are a component of the lancet magazine (21).

13. Lancet device according to one of the claims 10 to 12, **characterized in that** the magazine holder comprises a movable magazine carrier wherein the counter elements (23, 23a) are a component of the movable magazine carrier.

14. Lancet device according to one of the claims 10 to 13, **characterized in that** the movable gripper (22) of the step-switching mechanism does not engage with the counter elements (23, 23a) in the passive operating state of the magazine advancing mechanism.

15. Withdrawal system for withdrawing a sample of a body fluid comprising a lancet device (10) according to one of the previous claims and a lancet magazine (21) suitable therefor with several lancets.

## Revendications

1. Dispositif à lancettes (10) pour produire une piqûre, comprenant
- un système de réception de magasin pour un magasin à lancettes (21) contenant plusieurs lancettes ;
**caractérisé par**
- un entraînement de lancette avec un ressort d'entraînement, un moyen de tension pour tendre le ressort d'entraînement et un élément de liaison qui peut être mis en contact avec une lancette placée dans le magasin à lancettes (21) et qui au moyen de l'entraînement de lancette peut entraîner en mouvement la lancette mise en contact avec lui dans un mouvement de piqûre pour produire une piqûre ;
- un système d'avancement de magasin qui permet de faire avancer le magasin à lancettes (21) de sorte que les lancettes rangées dans celui-ci puissent être successivement mises en contact avec l'élément de liaison, et qui présente un état de fonctionnement actif et un état de fonctionnement passif, et
- un élément de sélection (17) qui permet de mettre le système d'avancement de magasin au choix dans l'état de fonctionnement actif ou dans l'état de fonctionnement passif,
dans l'état de fonctionnement actif, le système d'avancement de magasin faisant avancer automatiquement le magasin à lancettes (21) avant ou après un mouvement de piqûre de sorte que lors du mouvement de piqûre suivant, une lancette encore inutilisée du magasin à lancettes (21) est mise en contact avec l'élément de liaison de l'entraînement de lancette, et dans l'état de fonctionnement passif, le système d'avancement automatique de magasin étant désactivé de sorte que le magasin à lancettes (21) n'avance pas avant ou après un mouvement de piqûre d'une lancette, si bien que lors du mouvement de piqûre suivant, la même lancette que lors du mouvement de piqûre précédent est remise en contact avec l'élément de liaison de l'entraînement de lancette.

2. Dispositif à lancettes selon la revendication 1, **caractérisé en ce que** l'élément de liaison peut être mis en contact avec une lancette placée dans le magasin à lancettes (21) de telle sorte qu'il puisse être accouplé à la lancette placée dans le magasin à lancettes (21) et être entraîné dans un mouvement de piqûre au moyen de l'entraînement de lancette, conjointement avec la lancette accouplée à celui-ci, pour produire une piqûre.

3. Dispositif à lancettes selon la revendication 1 ou 2, **caractérisé en ce que** l'élément de liaison est conçu sous forme de tige de poussée.

4. Dispositif à lancettes selon l'une des revendications précédentes, **caractérisé en ce que** l'élément de sélection (17) est conçu de telle façon que le système d'avancement de magasin puisse être mis, chaque fois de manière réversible, dans l'état de fonctionnement actif ou passif.

5. Dispositif à lancettes selon l'une des revendications précédentes, **caractérisé en ce que** l'élément de sélection (17) est conçu de telle façon que le système d'avancement de magasin, après l'actionnement de l'élément de sélection (17), se trouve dans l'état de fonctionnement sélectionné jusqu'à ce que l'autre état de fonctionnement respectif soit sélectionné par un autre actionnement de l'élément de sélection.

6. Dispositif à lancettes selon l'une des revendications précédentes, **caractérisé en ce que** l'élément de sélection (17) est conçu de telle façon que le système d'avancement de magasin ne puisse passer d'un des deux états de fonctionnement à l'autre état de fonctionnement respectif que durant l'actionnement de l'élément de sélection (17).

7. Dispositif à lancettes selon l'une des revendications précédentes, **caractérisé en ce que** dans l'état de fonctionnement actif, le système d'avancement de magasin est couplé mécaniquement à l'entraînement de lancette (25).

8. Dispositif à lancettes selon l'une des revendications précédentes, **caractérisé en ce que** dans l'état de fonctionnement actif, le système d'avancement de magasin est couplé au moyen de tension, de telle sorte que l'avancement du magasin à lancettes (21) a lieu pendant l'actionnement du moyen de tension servant à tendre le ressort d'entraînement.

9. Dispositif à lancettes selon l'une des revendications précédentes, **caractérisé en ce que** le système d'avancement de magasin comprend un mécanisme pas à pas (20).

10. Dispositif à lancettes selon la revendication 9, **caractérisé en ce que** le mécanisme pas à pas (20) comprend un élément de préhension mobile (22) ainsi que plusieurs contre-éléments (23, 23a), l'élément de préhension mobile (22) étant disposé de telle façon qu'il puisse être mis en prise avec les contre-éléments, ce qui fait avancer le magasin à lancettes (21).

11. Dispositif à lancettes selon la revendication 9 ou 10, **caractérisé en ce que** l'élément de préhension mobile (22) du mécanisme pas à pas (20) fait partie du dispositif à lancettes (10).

12. Dispositif à lancettes selon l'une des revendications 10 ou 11, **caractérisé en ce que** les contre-éléments (23, 23a) font partie du magasin à lancettes (21).

13. Dispositif à lancettes selon l'une des revendications 10 à 12, **caractérisé en ce que** le système de réception de magasin comprend un support de magasin mobile, les contre-éléments (23, 23a) faisant partie du support de magasin mobile.

14. Dispositif à lancettes selon l'une des revendications 10 à 13, **caractérisé en ce que** dans l'état de fonctionnement passif du système d'avancement de magasin, l'élément de préhension mobile (22) du mécanisme pas à pas ne vient pas en prise avec les contre-éléments (23, 23a).

15. Système de prélèvement destiné à prélever un échantillon d'un fluide corporel, comprenant un dispositif à lancettes (10) selon l'une des revendications précédentes et un magasin à lancettes (21) adapté contenant plusieurs lancettes.
